# EUROPEAN PATENT APPLICATION

(11) **EP 3 912 543 A1**
(43) Date of publication of application: **24.11.2021**
(21) Application number: 21175289.4
(22) Date of filing: 21.05.2021
(51) Int. Cl.: A61B 3/00

(54) **OPHTHALMIC UNIT WITH A SYSTEM FOR AUTOMATICALLY POSITIONING THE PATIENT'S CHAIR RELATIVE TO THE INSTRUMENT-CARRYING TABLE**

(30) Priority: 22.05.2020 IT 202000012067
(71) Applicant: Frastema Ophthalmics Easyopht Group S.r.l. (in forma abbreviata FE - Group S.r.l.), 21052 Busto Arsizio (VA) (IT)
(72) Inventor: Carnaghi, Matteo, 21052 Busto Arsizio VA (IT)
(74) Representative: Rondano, Davide

(57) **Abstract**

The ophthalmic unit (10) comprises a chair (12) for a patient and an instrument-carrying table (16) carrying an ophthalmic instrument (18) and a support device (24) for supporting the head of the patient. The chair (12) and the table (16) are each supported so as to be vertically adjustable between a respective position of minimum height from the ground (hₘᵢₙ, Hₘᵢₙ) and a respective position of maximum height from the ground (hₘₐₓ, Hₘₐₓ). The ophthalmic unit (10) is provided with an automatic positioning system for automatically adjusting the vertical position of the chair (12) relative to the table (16). The automatic positioning system comprises a first linear actuation device (30) arranged to vertically move the chair (12), a second linear actuation device (32) arranged to vertically move the table (16), an electronic control unit arranged to automatically control, according to predetermined operating logics, the first linear actuation device (30) and/or the second linear actuation device (32) to adjust the vertical position of the chair (12) relative to the table (16), and a first presence sensor (42) arranged to provide the electronic control unit with information relating to the presence of the patient sitting on the chair (12) at a given first height from the ground (Hi).

## Description

### Technical field of the invention

The present invention relates in general to the field of the apparatuses for ophthalmic use. More specifically, the present invention relates to an ophthalmic unit provided with an automatic positioning system for automatically positioning an instrument-carrying table relative to a patient's chair.

### State of the art

An ophthalmic unit typically comprises an instrument-carrying table (hereinafter simply referred to, for the sake of convenience, as table) and a chair for the patient (hereinafter simply referred to, for the sake of convenience, as chair), on which the patient remains seated during an eyesight test. The table carries one or more instruments normally used by the eye doctor to test the eyesight of a patient, such as a tonometer, an ophthalmometer, an auto refractometer, a slit lamp, etc. The table also carries one or more devices for supporting the head of the patient, each having a forehead rest and a chin rest, typically one support device for each ophthalmic instrument mounted on the table. Both the table and the chair are typically provided with respective motorized adjustment devices by means of which the operator (i.e. the eye doctor) is able to adjust the vertical position of the table and of the chair independently of each other. By acting on suitable control members, such as for example (physical or virtual) push buttons, the operator is thus able to control the upward or downward vertical movement of the table and/or of the chair so as to ensure the correct positioning of the eyes of the patient seated on the chair relative to the instruments mounted on the table, that is, the alignment of the visual axis of the patient with the optical axis of the instrument that is being used each time for the eyesight test.

Ophthalmic units are also known, which are provided with automatic positioning systems for automatically adjusting the position of the table relative to the chair.

In this connection, CN 206355285 U discloses an eyesight test system comprising a chair for the eye doctor, a chair for the patient and a table with the diagnostic instruments, wherein both the chairs and the table are adjustable in height, independently of each other, by means of suitable electrically-operated adjustment mechanisms. The positioning of the chairs and of the table is automatically controlled by a control unit based on the identification of the eye doctor and of the patient, the control unit having stored in advance the optimal positions of the chairs and of the table for that specific eye doctor and that specific patient. This known system therefore requires identification means for identifying the identity of the eye doctor and of the patient, as well as storing means for storing data associated to each eye doctor and each patient. Accordingly, in addition to being particularly complex, this known system is not able to adjust automatically the positions of the chairs and of the table in case the data of a given eye doctor and/or of a given patient have not been previously stored in the storing means of the system.

Furthermore, US 9 351 635 discloses a system and a method for automatically positioning an ophthalmic apparatus by automatic control, based on information relating to the 3D geometry of the face and of the body of the patient, of actuating units that control the movement of the patient's chair and/or of the instrument-carrying table. This known automatic positioning system requires therefore a scanning apparatus arranged to acquire the 3D geometry of the face and of the body of the patient, thereby resulting to be quite complex and expensive.

### Summary of the invention

It is an object of the present to provide an ophthalmic unit provided with an automatic positioning system for automatically adjusting, depending on the height of the patient, the position of the table relative to the chair so as to ensure the correct positioning of the patient relative to the instruments mounted on the table, which is less complex and expensive than the prior art discussed above.

This and other objects are fully achieved by virtue of an ophthalmic unit having the features set forth in independent claim 1.

Preferred embodiments of the ophthalmic unit according to the present invention are the subject-matter of the dependent claims.

In short, the invention is based on the idea of using an automatic positioning system comprising a first linear actuation device arranged to vertically move the chair between a position of minimum height from the ground and a position of maximum height from the ground, a second linear actuation device arranged to vertically move the table between a position of minimum height from the ground and a position of maximum height from the ground, a first presence sensor arranged to detect the presence or absence of the patient sitting on the chair at a given first height from the ground, and an electronic control unit arranged to automatically carry out a method for positioning the table and the chair, by suitably controlling the first and/or the second actuation device based on the information provided by the first presence sensor about the presence or absence of the patient at said first height from the ground.

The first height from the ground is chosen as the height at which the highest point of the head of the patient is placed when the patient has his eyes aligned with the optical axis of the instrument in the position of maximum height of the table from the ground. The first height from the ground will therefore be at a given distance above the forehead rest of the support device mounted on the table when the table is in the position of maximum height from the ground.

In this case, the automatic positioning method is for example carried out starting from an initial operating condition in which the chair is in the position of minimum height from the ground, while the table is in the position of maximum height from the ground, and checking if the first presence sensor intercepts the patient sitting on the chair. If not, the chair is moved upwards until the first presence sensor intercepts the patient or the chair reaches the position of maximum height from the ground.

Preferably, the ophthalmic unit further comprises a second presence sensor arranged to detect the presence or absence of the patient sitting on the chair at a given second height from the ground smaller than the first height from the ground.

The aforementioned second height from the ground is conveniently equal to the height at which the highest point of the head of the patient is placed when the patient has his eyes aligned with the optical axis of the instrument in the position of minimum height of the table from the ground. The second height from the ground will therefore be at a given distance above the forehead rest of the support device mounted on the table when the table is in the position of minimum height from the ground.

In this case, the automatic positioning method controlled by the electronic control unit provides that, once the chair and the table have been moved to the aforementioned initial operating condition, the presence of the patient at the height corresponding to the position of the second presence sensor is checked by means of that sensor. In case the presence is confirmed, if the first presence sensor does not intercept the patient sitting on the chair at the height corresponding to the position of that sensor, then the chair is moved upwards until the first presence sensor intercepts the patient or the chair reaches the position of maximum height from the ground. In case the presence is not confirmed, the table is moved to the position of minimum height from the ground and the chair is moved upwards until the second presence sensor intercepts the patient or the chair reaches the position of maximum height from the ground.

The first and/or the second presence sensor are advantageously direct reflection type photoelectric sensors.

By virtue of the fact that the automatic positioning system provides only for one or at most two presence sensors, it is less complex and expensive than the prior art discussed above. Moreover, the automatic positioning method can be carried out in less time than the prior art, as it does not require a 3D scanning of the face and of the body of the patient. Additionally, the positioning system is able to work without the need to store in advance the data relating to the patient and therefore does not require storing means for storing these data.

The ophthalmic unit is also provided with control means operable by the operator, once the table and the chair have been automatically positioned, to adjust the height of the table depending on his own position. These control means are operatively connected to the electronic control unit, which is configured to send suitable control signals to the first and the second actuation device, based on the commands imparted by the operator through the aforementioned control means, to vertically move not only the table, but also the chair, so as to keep the table and the chair at the same relative distance previously set during the automatic positioning procedure.

Once the table and the chair have been positioned according to the aforementioned procedure, the operator may adjust the position of the table to be as comfortable as possible for him, without thereby changing the relative position, set by the automatic positioning system, of the table with respect to the chair.

The ophthalmic unit according to the present invention allows therefore not only to automatically position in the correct position the patient relative to the instruments mounted on the table, but also to adjust the table, and hence the instruments mounted thereon, in the most comfortable position for the operator, without the risk that this latter adjustment adversely affects the automatic adjustment previously carried out.

The aforementioned control means may include a joystick placed on the table or on a cabinet of the ophthalmic unit supporting the table. Alternatively, or additionally, the aforementioned control means may include a touchscreen placed on the cabinet.

The touchscreen may be configured to allow not only the manual adjustment of the height of the table (and, along with the same, of the chair), but also to control additional functions of the unit, such as for example the automatic positioning procedure, the switching on and off of the instruments mounted on the table, etc.

Preferably, the ophthalmic unit comprises a support pole on which the first sensor and (if present) the second sensor are mounted.

The support pole may also act as support for LED lights and/or further accessories, if any, of the ophthalmic unit.

Further features of the present invention will become apparent from the following detailed description, given purely by way of non-limiting example.

### Brief description of the drawings

In the following detailed description of the invention reference will be made to the enclosed drawings, wherein:
- Figure 1 is a perspective view of an ophthalmic unit according to an embodiment of the present invention,
- Figure 2 is a side view showing, in addition to the ophthalmic unit of Figure 1, a chair for a operator,
- Figure 3 schematically shows the adjustment ranges for the vertical position of the chair and of the table of the ophthalmic unit of Figure 1;
- Figure 4 is a block diagram illustrating the automatic procedure for vertically positioning the chair and the table of the unit of Figure 1;
- Figure 5 is a block diagram illustrating the automatic procedure for vertically positioning the chair and the table in another embodiment of the ophthalmic unit according to the present invention; and
- Figures 6 and 7 show respective screens of a touchscreen of the ophthalmic unit of Figure 1.

### Detailed description of preferred embodiments

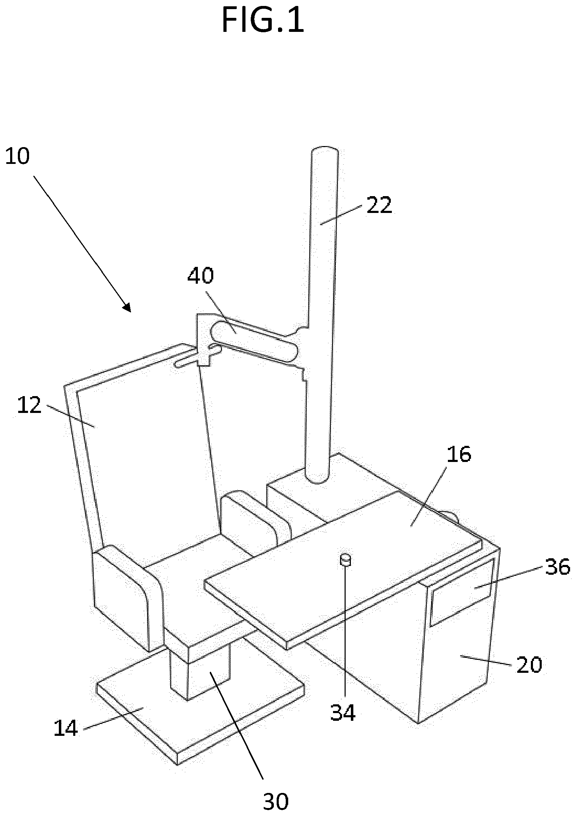

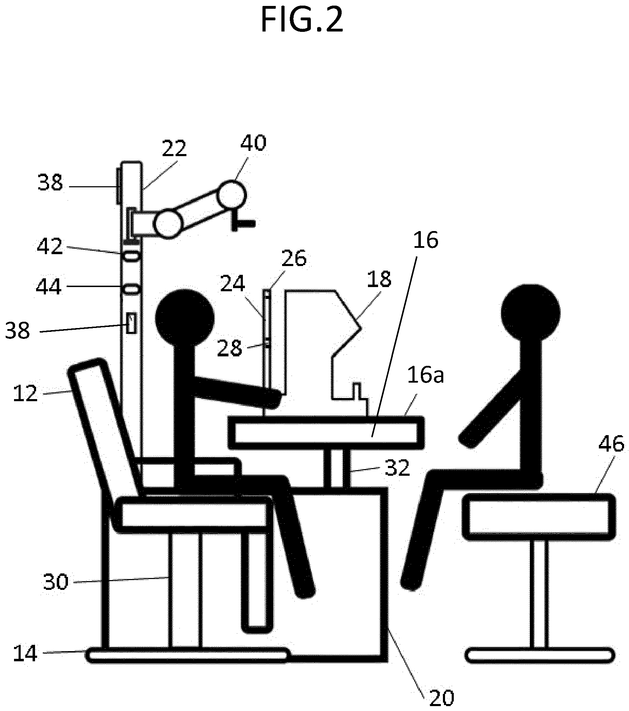

With reference first to Figures 1 and 2, an ophthalmic unit according to an embodiment of the present invention is generally indicated 10.

The ophthalmic unit 10 basically comprises a chair 12 for the patient, a base 14 supporting the chair 12, an instrument-carrying table 16 on which an ophthalmic instrument 18 (only schematically shown, as it is of per-se-known type), such as for example a tonometer, an ophthalmometer, an auto refractometer, a slit lamp, etc., is mounted, a cabinet 20 supporting the table 16, and a support pole 22 carried by the cabinet 20.

In the example of Figures 1 and 2, the table 12 carries only one instrument 18, but of course more instruments might be provided for, without thereby departing from the scope of the present invention. In a per-se-known manner, a support device 24 for supporting the head of the patient is associated to the instrument 18. The support device 24 comprises a forehead rest 26 and a chin rest 28 suitably arranged so as to correctly position the head of the patient, with the forehead in abutment against the forehead rest 26 and with the chin resting on the chin rest 28, relative to the instrument 18. The support device 24 may be mounted on the table 16 or integrated in the instrument 18. Irrespective of the way the support device 24 is mounted, the forehead rest 26 will be at a given height (for example 385 mm) relative to a support plane 16a of the table 16. Both the chair 12 and the table 16 are adjustable vertically, each between a respective position of minimum height from the ground and a respective position of maximum height from the ground. More specifically, the chair 12 is adjustable vertically by means of a linear actuation device 30 (only schematically shown in Figures 1 and 2) interposed between the chair 12 and the base 14, while the table 16 is adjustable vertically by means of a linear actuation device 32 (only schematically shown in Figures 1 and 2) interposed between the table 16 and the cabinet 20. The linear actuation devices 30 and 32 may be of any known type, for example electromechanical actuators comprising an electric motor and a motion conversion mechanism (such as a screw and nut mechanism) for converting the rotary motion of the electric motor into a translational motion. The linear actuation devices 30 and 32 are operatively connected to an electronic control unit (contained in the cabinet 20) to be controlled by the latter so as to cause the upward or downward vertical movement of the chair 12 and of the table 16, respectively.

As will be explained in detail further on, the linear actuation devices 30 and 32 are operated by the electronic control unit in automatic mode, based on predetermined control logics, or in manual mode, based on commands imparted by the operator through suitable control means. Said control means comprise for example a joystick 34 mounted on the table 16, through which the operator can control the vertical movement of the chair 12 and/or of the table 16. Preferably, said control means further comprise virtual buttons shown on a touchscreen 36 mounted on the cabinet 20.

The support pole 22 carries one or more lights 38, preferably made as LED lights and preferably having an adjustable light intensity. Moreover, an orientable support arm 40 is preferably mounted on the support pole 22 for supporting an ophthalmic instrument, such as for example a phoropter.

The ophthalmic unit 10 further comprises a first presence sensor 42 and a second presence sensor 44, each of which is mounted at a respective height from the ground, in particular on the support pole 22, and is operatively connected to the electronic control unit to provide information relating to the presence/absence of the patient at the height the sensor is positioned. More specifically, the first presence sensor 42 is positioned at a height from the ground corresponding to the highest point alto of the head of the patient when the latter has his eyes aligned with the optical axis of the instrument 18 in the position of maximum height of the table 16from the ground, while the second presence sensor 44 is positioned at a height from the ground corresponding to the highest point of the head of the patient when the latter has his eyes aligned with the optical axis of the instrument 18 in the position of minimum height of the table 16 from the ground. The first presence sensor 42 will therefore be positioned at a height exceeding by a certain distance D (equal for example to 100 mm, but more generally comprised between 0 mm and 200 mm, preferably between 50 mm and 150 mm) the height at which the forehead rest 26 of the support device 24 is positioned when the table 16 is in the position of maximum height from the ground, while the second presence sensor 44 will be positioned at a height exceeding by said distance D the height at which the forehead rest 26 of the support device 24 is positioned when the table 16 is in the position of minimum height from the ground. The first presence sensor 42 and the second presence sensor 44 are for example made as photoelectric sensors, preferably direct reflection type photoelectric sensors.

Finally, numeral 46 in Figure 2 indicates a chair for the operator (i.e. the eye doctor), on which the operator remains seated to carry out the eyesight test of the patient sitting on the chair 12.

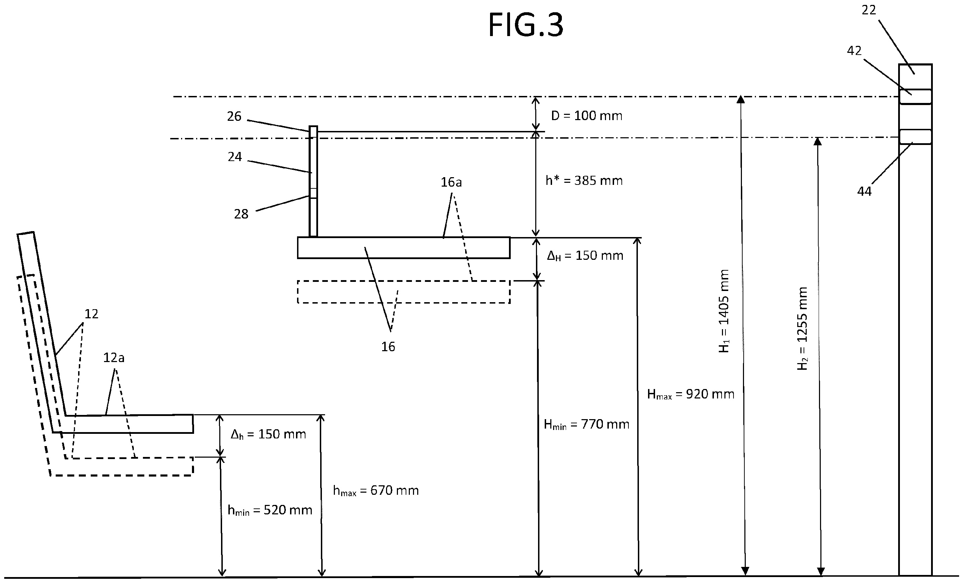

Figure 3 schematically show, purely by way of non-limiting examples, the ranges of adjustment of the vertical position of the chair 12 and of the table 16 (which are both shown in dashed line in the position of minimum height and in continuous line in the position of maximum height), as well as the vertical arrangement of the presence sensors 42 and 44.

As far as the chair 12 is concerned, the position of minimum height from the ground (referred to the sitting plane, indicated 12a, of the chair) is at a height hₘᵢₙ equal to 520 mm, while the position of maximum height from the ground is at a height hₘₐₓ equal to 670 mm, assuming a range of adjustment Δₕ of the chair 12 equal to 150 mm. With the chair 12 in the position of minimum height from the ground, a person with a height equal for example to 192 cm (corresponding to the 99 percentile of the Italian population) sitting on the chair 12 will have his eyes positioned at a height from the sitting plane 12a approximately equal to 800 mm and therefore at a height from the ground approximately equal to 1320 mm. Considering a height h^{∗} of the forehead rest 26 of the support device 24 equal to 385 mm with respect to the support plane 16a of the table 16, in order to allow the correct positioning of the eyes of a patient of that height relative to the instrument 18 the table 16 (or, better, the support plane 16a of the same) will have to be at a height from the ground approximately equal to 920 mm.

Considering that height from the ground as the maximum height Hₘₐₓ and assuming for the table 16 a range of adjustment Δ_{H} equal to that of the chair 12, namely 150 mm, the position of minimum height from the ground of the table 16 will be at a height Hₘᵢₙ equal to 770 mm. With these ranges of adjustment for the chair 12 and the table 16 it is possible to position in the correct position any patients, from a 7-year-old boy (whose eyes are positioned, in the seated condition, at a height which is on average approximately equal to 560 mm relative to the sitting plane 12a of the chair 12) up to an adult with a height equal to 192 cm.

The presence sensors 42 and 44 are vertically positioned, as explained above, depending on the positions of maximum and minimum height of the table 16from the ground, namely at the height H₁ = Hₘₐₓ + h^{∗} + D and at the height H₂ = Hₘᵢₙ + h^{∗} + D, respectively. In the embodiment proposed herein, therefore, the height H₁ of the first presence sensor 42 will be equal to 1405 mm (920 mm + 385 mm + 100 mm), while the height H₂ of the second presence sensor 44 will be equal to 1255 mm (770 mm + 385 mm + 100 mm). Accordingly, when the patient sitting on the chair 12 will be below the height H₂, his presence will not be detected either by first presence sensor 42 or by the second presence sensor 44, whereas when the patient sitting on the chair 12 will be at a height comprised between H₂ and H₁, his presence will be detected only by the second presence sensor 44, and finally when the patient sitting on the chair 12 will be above the height H₁, his presence will be detected both by the first presence sensor 42 and by the second presence sensor 44.

As previously stated, the electronic control unit is configured to automatically control the linear actuation devices 30 and 32 to vertically move the chair 12 and the table 16, respectively, in order to ensure the correct positioning of the patient sitting on the chair 12 relative to the instrument 18 mounted on the table 16. In order for the eyesight test to be correctly performed, it is in fact important that the visual axis of the eyes of the patient is as aligned as possible with the optical axis of the instrument 18, and that therefore the patient rests with his head on the support device 24 keeping a correct posture, without for example leaning forward because he is positioned at an excessive height relative to the support device 24.

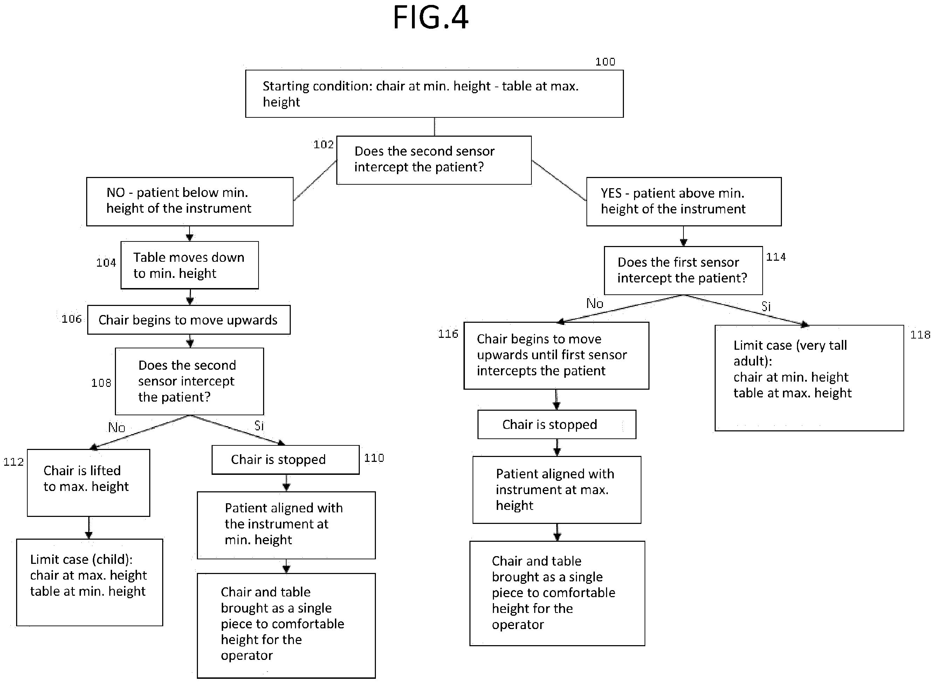

With reference to the block diagram of Figure 4, it is now illustrated the positioning procedure that is carried out automatically, under control of the electronic control unit, once the patient is seated on the chair 12 and the operator has given a start command for starting the procedure (for example by touching a special virtual button on the touchscreen 36).

First of all, the electronic control unit controls the linear actuation devices 30 and 32 to bring the chair 12 and the table 16 in an initial operating condition in which the chair 12 is at the minimum height hₘᵢₙ while the table 16 is at the maximum height Hₘₐₓ (block 100).

It is then checked (block 102) whether the second presence sensor 44 intercepts the patient sitting on the chair 12 or not, i.e. whether the patient is at a height greater or smaller than the height H₂ of the second sensor associated to the condition of minimum height of the instrument 18 from the ground.

If not (patient below the height H₂), the table 16 is brought at the minimum height Hₘᵢₙ (block 104), and then the chair 12 begins to be lifted (block 106).

While the chair 12 is being lifted, the second presence sensor 44 continues to check the presence of the patient at the relating height H₂ (block 108).

As soon as the second presence sensor 44 intercepts the patient, the electronic control unit stops the upward movement of the chair 12 (block 110). In the position thus reached, the patient is therefore correctly positioned relative to the instrument 18 with the table 16 at the minimum height Hₘᵢₙ. If, on the other hand, the second presence sensor 44 does not intercept the patient, since the patient has a very low height, the chair 12 continues to be lifted until it reaches the maximum height hₘₐₓ (block 112).

If the check that is done at block 102 is positive (patient above the height H₂), then the electronic control unit checks (block 114) whether the first presence sensor 42 intercepts the patient or not, i.e. whether the patient is at a height greater or smaller than the height H₁ of the first sensor associated to the maximum height of the instrument 18 from the ground.

If not (patient below the height H₁), the chair 12 is lifted until when the first presence sensor 42 intercepts the patient (block 116). In the position thus reached, the patient is therefore correctly positioned relative to the instrument 18 with the table 16 at the maximum height Hₘₐₓ.

If yes (patient below the height H₁, i.e. very tall patient), both the chair 12 and the table 16 are kept in the aforementioned initial operating condition (block 118).

With the procedure illustrated above, the ophthalmic unit 10 is able to automatically adjust the position of the patient sitting on the chair 12 relative to the instrument 18 mounted on the table 16 in a range of adjustment equal to the sum of the ranges of adjustment of the chair and of the table (in the example proposed herein, a range of 300 mm).

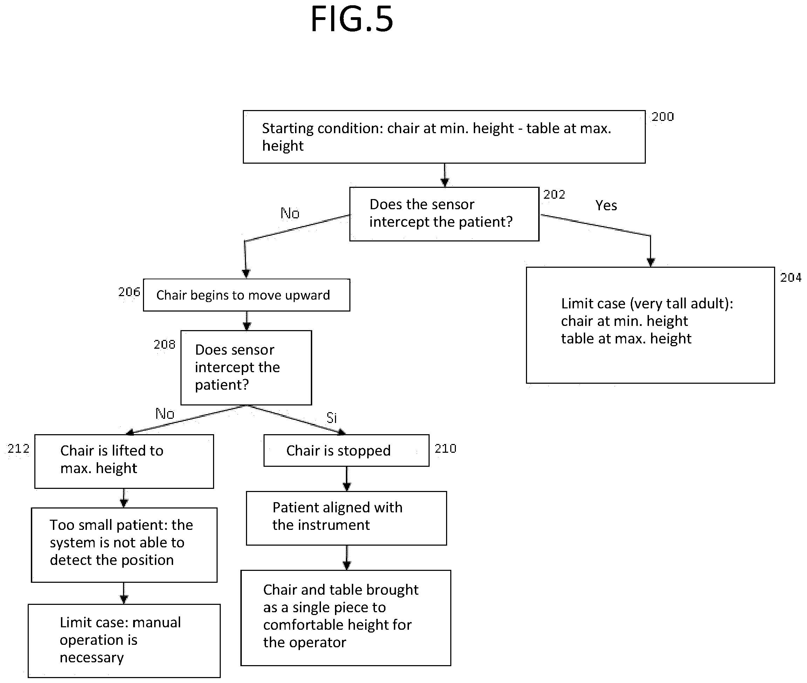

The block diagram of Figure 5 illustrates the automatic positioning procedure in case of an ophthalmic unit which differs from the one described above in that it comprises only one sensor, instead of two sensors, namely only the first presence sensor 42. Also in this case the first presence sensor 42 is preferably positioned at a height H₁ exceeding by the distance D (also in this case equal to 100 mm) the height (1305 mm) at which the forehead rest 26 of the support device 24 is positioned when the table 16 is at the maximum height from the ground Hₘₐₓ. Alternatively, the first presence sensor 42 might be positioned at a height smaller than the one indicated above.

The positioning procedure provides also in this case for an initial operating condition in which the chair 12 is at the minimum height hₘᵢₙ while the table 16 is at the maximum height Hₘₐₓ (block 200).

It is then checked (block 202) whether the presence sensor 42 intercepts the patient sitting on the chair 12.

If yes (patient above the height H₁, i.e. very tall patient), both the chair 12 and the table 16 are kept in the aforementioned initial operating condition (block 204).

If not (patient below the height H₁), the electronic control unit causes, by means of the linear actuation device 30, the lifting of the chair 12 (block 206).

While the chair 12 is moving upwards, the presence sensor 42 continues to check the presence of the patient at the relating height H₁ (block 208).

As soon as the presence sensor 42 intercepts the patient, the electronic control unit stops the upward movement of the chair 12 (block 210). In the position thus reached, the patient is therefore correctly positioned relative to the instrument 18 with the table 16 at the maximum height Hₘₐₓ. If, on the other hand, the presence sensor 42 does not intercept the patient, because he has a too small height, the chair 12 continues to be lifted until it reaches the maximum height hₘₐₓ (block 212). At that point the operator may in any case adjust manually the vertical position of the table 16 to adapt it to the position of the patient.

In case only one presence sensor is used, the ophthalmic unit 10 is able to adjust automatically the position of the patient sitting on the chair 12 relative to the instrument 18 mounted on the table 16 in a range of adjustment only equal to the range of adjustment of the chair (in the proposed example, a range of 150 mm). Although the range of adjustment is smaller, in the automatic positioning mode, the ophthalmic unit 10 is in this case less expensive as it requires only one presence sensor instead of two. Both in case only one presence sensor is provided for and in case two presence sensors are provided for, the ophthalmic unit 10 according to the present invention additionally offers the possibility to adjust, once the automatic positioning method described above has been carried out, the vertical position of the table 16, and hence of the instrument 18, relative to the operator sitting on the chair 46.

This adjustment may be done manually by the operator through the joystick 34 or the touchscreen 36. In this connection, the electronic control unit is configured to control, based on of the inputs received from the operator, not only the linear actuation device 32 associated to the table 16, but also the linear actuation device 30 associated to the chair 12, so as to ensure maintenance of the position of the table 16 relative to the chair 12 which has been set with the automatic positioning method previously carried out. In this manner, the ophthalmic unit allows the operator to adjust the vertical position of the table 16, and hence of the instrument 18, which is the most comfortable one for him, without thereby changing the position of the patient relative to the table 16, and hence relative to the instrument 18 and the support device 24 mounted on the table 16.

Advantageously, the final adjustment of the table 16 (and, along with it, the corresponding displacement of the chair 12) may also be carried out automatically, based for example on a preferred height stored in advance in a memory of the electronic control unit. In this case, therefore, once the automatic positioning of the table 16 relative to the chair 12 has been completed, the electronic control unit will control the movement of the table 16 and of the chair 12, as a single piece with each other, in order to bring the table 16 to the height established by the operator (naturally, insofar as this is allowed by vertical adjustment ranges of the chair 12 and of the table 16).

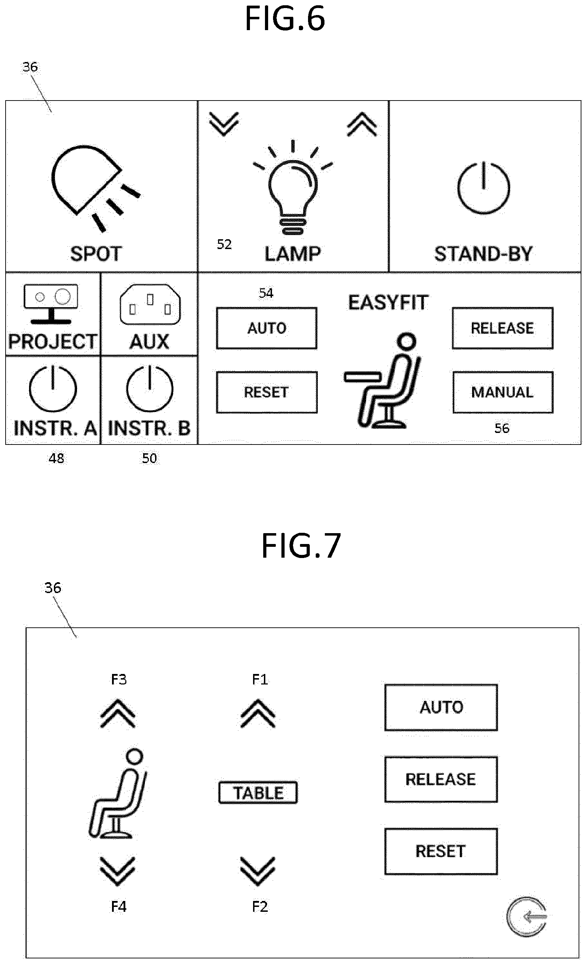

Figures 6 and 7 finally show two examples of screens of the touchscreen 36 by means of which the operator is able to control the operation of the ophthalmic unit 10.

The screen of Figure 6 shows the main menu, with the main virtual buttons for controlling a given device or activating a given function. For example, by means of the virtual button indicated at 48 the operator may switch on/off the instrument 18 mounted on the table 16, by means of the virtual button indicated at 50 the operator may switch on/off a further instrument 18, if any, mounted on the table 16, by means of the virtual button indicated at 52 the operator may adjust the intensity of the lights 38, by means of the virtual button indicated at 54 the operator may activate the automatic positioning method and by means of the virtual button indicated at 56 the operator may activate the function of manual adjustment of the vertical position of the table and of the chair. When the latter button is touched, the screen shown in Figure 7 appears on the touchscreen 36, wherein the operator may control manually the upward or downward movement of the chair and of the table touching the arrows F1 and F2 associated to the table and the arrows F3 and F4 associated to the chair, respectively.

Naturally, the layout of the screens, as well as the arrangement and the aspect of the virtual buttons, may differ from those shown in Figures 6 and 7.

The present invention has been disclosed with reference to a preferred embodiment thereof. It is clear that other embodiments may be envisaged, which share with the one described herein the same inventive concept, as defined by the scope of the enclosed claims.

## Claims

1. Ophthalmic unit (10) comprising a chair (12) for a patient and an instrument-carrying table (16) carrying an ophthalmic instrument (18) and a support device (24) for supporting the head of the patient,
wherein the chair (12) and the table (16) are each supported so as to be vertically adjustable between a respective position of minimum height from the ground (hₘᵢₙ, Hₘᵢₙ) and a respective position of maximum height from the ground (hₘₐₓ, Hₘₐₓ), wherein the support device (24) comprises a forehead rest (26) placed at a given height (h^{∗}) relative to a support plane (16a) of the table (16), and
wherein the ophthalmic unit (10) further comprises an automatic positioning system for automatically adjusting the vertical position of the chair (12) relative to the table (16) to ensure the correct positioning of the eyes of the patient sitting on the chair (12) relative to the ophthalmic instrument (18) mounted on the table (16), said automatic positioning system comprising a first linear actuation device (30) arranged to vertically move the chair (12) between the respective positions of minimum height from the ground (hₘᵢₙ) and of maximum height from the ground (hₘₐₓ), a second linear actuation device (32) arranged to vertically move the table (16) between the respective positions of minimum height from the ground (Hₘᵢₙ) and of maximum height from the ground (Hₘₐₓ), and an electronic control unit arranged to automatically control, according to predetermined operating logics, said first linear actuation device (30) and/or said second linear actuation device (32) to adjust the vertical position of the chair (12) relative to the table (16),
**characterized**
**in that** the automatic positioning system further comprises a first presence sensor (42) arranged to provide the electronic control unit with information relating to the presence of the patient sitting on the chair (12) at a given first height from the ground (H₁), and in that the electronic control unit is arranged to control the first linear actuation device (30) and/or the second linear actuation device (32) based on of the information provided by said first presence sensor (42).

2. Ophthalmic unit according to claim 1, wherein said first presence sensor (42) is a direct-reflection photoelectric sensor.

3. Ophthalmic unit according to claim 1 or claim 2, wherein said first height from the ground (Hi) exceeds by a given distance (D) the height from the ground (h^{∗}+Hₘₐₓ) at which the forehead rest (26) of the support device (24) is positioned when the table (16) is in the position of maximum height from the ground (Hₘₐₓ).

4. Ophthalmic unit according to claim 3, wherein said distance (D) is comprised between 0 mm and 200 mm, preferably between 50 mm and 150 mm, more preferably equal to 100 mm.

5. Ophthalmic unit according to any one of the preceding claims, wherein the electronic control unit is programmed to carry out the automatic positioning of the chair (12) and of the table (16) according to the following steps:
a) initially bringing the chair (12) to the respective position of minimum height from the ground (hₘᵢₙ) and the table (16) to the respective position of maximum height from the ground (Hₘₐₓ); and
b) checking, by means of said first presence sensor (42), whether the patient sitting on the chair (12) is present at said first height from the ground (H₁) and, if not, moving the chair (12) upwards until said first presence sensor (42) detects the presence of the patient at said first height from the ground (H₁) or the chair (12) reaches the respective position of maximum height from the ground (hₘₐₓ).

6. Ophthalmic unit according to any one of claims 1 to 4, wherein the automatic positioning system further comprises a second presence sensor (44) arranged to provide the electronic control unit with information relating to the presence of the patient sitting on the chair (12) at a given second height from the ground (H₂) smaller than said first height from the ground (H₁), and wherein the electronic control unit is arranged to control the first linear actuation device (30) and/or the second linear actuation device (32) depending not only on the information provided by said first presence sensor (42), but also on the information provided by said second presence sensor (44).

7. Ophthalmic unit according to claim 6, wherein said second presence sensor (44) is a direct-reflection photoelectric sensor.

8. Ophthalmic unit according to claim 6 or claim 7, wherein said second height from the ground (H₂) exceeds by said distance (D) the height from the ground (h^{∗}+Hₘᵢₙ) at which the forehead rest (26) of the support device (24) is positioned when the table (16) is in the position of minimum height from the ground (Hₘᵢₙ).

9. Ophthalmic unit according to any one of claims 6 to 8, wherein the electronic control unit is programmed to carry out the automatic positioning of the chair (12) and of the table (16) according to the following steps:
a) initially bringing the chair (12) to the respective position of minimum height from the ground (hₘᵢₙ) and the table (16) to the respective position of maximum height from the ground (Hₘₐₓ);
b) checking, by means of said second presence sensor (44), if the patient sitting on the chair (12) is present at said second height from the ground (H₂); and
c) if the checking at previous step b) is positive, then moving the chair (12) upwards until said first presence sensor (42) detects the presence of the patient at said first height from the ground (H₁) or the chair (12) reaches the respective position of maximum height from the ground (hₘₐₓ); or
d) if the checking at previous step b) is negative, bringing the table (16) to the respective position of minimum height from the ground (Hₘᵢₙ) and moving the chair (12) upwards until said second presence sensor (44) detects the presence of the patient or the chair (12) reaches the respective position of maximum height from the ground (hmax).

10. Ophthalmic unit according to any one of the preceding claims, further comprising control means (34, 36), in particular a joystick (34) and a touchscreen (36), operable by the operator to manually control said first linear actuation device (30) and/or said second linear actuation device (32).

11. Ophthalmic unit according to any one of the preceding claims, wherein the electronic control unit is programmed to control the vertical movement of the chair (12) and of the table (16), as a single piece with each other, once the automatic positioning of the chair (12) relative to the table (16) has been completed, to bring the table (16) at a given height from the ground which is manually adjustable by the operator or is set in advance by the operator.

12. Ophthalmic unit according to any one of the preceding claims, further comprising a support pole (22) on which said first presence sensor (42) is mounted.

13. Ophthalmic unit according to claim 12 when depending on any one of claims 6 to 11, wherein said second presence sensor (44) is also mounted on the support pole (22).

14. Ophthalmic unit according to claim 12 or claim 13, wherein the support pole (22) carries at least one light (38).

15. Ophthalmic unit according to any one of the preceding claims, wherein the difference between the maximum height from the ground (hₘₐₓ) and the minimum height from the ground (hₘᵢₙ) of the chair (12) is equal to the difference between the maximum height from the ground (Hₘₐₓ) and the minimum height from the ground (Hₘᵢₙ) of the table (16).
